(19) Europäisches Patentamt
European Patent Office
Office européen des brevets

(11) Veröffentlichungsnummer: **0 298 467**
**A2**

# (12) EUROPÄISCHE PATENTANMELDUNG

(21) Anmeldenummer: **88110818.7**

(22) Anmeldetag: **07.07.88**

(51) Int. Cl.4: **C07D 487/04 , A61K 31/505 , //(C07D487/04,249:00,239:00)**

(30) Priorität: **10.07.87 DE 3722802**

(43) Veröffentlichungstag der Anmeldung: **11.01.89 Patentblatt 89/02**

(84) Benannte Vertragsstaaten: **DE FR GB**

(71) Anmelder: **HOECHST AKTIENGESELLSCHAFT**
**Postfach 80 03 20**
**D-6230 Frankfurt am Main 80(DE)**

(72) Erfinder: **Winkelmann, Erhardt, Dr.**
**Brunhildenweg 5**
**D-6233 Kelkheim (Taunus)(DE)**
Erfinder: **Winkler, Irvin, Dr.**
**In den Eichen 40**
**D-6237 Liederbach(DE)**

(54) **In 6- und 9-Stellung substituierte 2-Amino-8-aza-purine, ihre Verwendung, diese Purine enthaltende Arzneimittel und Verfahren zur Herstellung der Purine.**

(57) Verbindungen der Formel 1

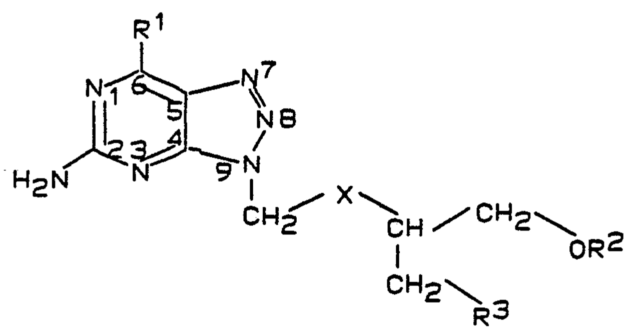

(1)

worin
$R^1$ H, Halogen, OH, SH, Azido, $NH_2$ oder Z-$R^5$ ist, wobei Z O, S, SO, $SO_2$ oder NH und $R^5$ einen acyclischen aliphatischen Kohlenwasserstoffrest mit 1 bis 6 C-Atomen bedeutet, welcher a) unsubstituiert ist oder b) durch wenigstens eines der Merkmale modifiziert ist, daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl, das direkt oder über eine Alkylengruppe von 1 bis 3 C-Atomen an Z gebunden ist, oder Phenyl darstellt, wobei der Phenylring jeweils durch Halogen, Trifluormethyl, Alkoxy oder Alkylthio mit jeweils 1 bis 3 C-Atomen substituiert sein kann,
$R^2$ H ist oder die Bedeutung von $R^5$ hat oder eine Acylgruppe CO-$R^6$ ist, wobei $R^6$ Alkyl mit 1 bis 6 C-Atomen, Benzyl oder Phenyl ist,
$R^3$ Halogen, Azido, unsubstituiertes Amino oder den Rest Y-$R^4$ bedeutet, wobei $R^4$ dieselbe Bedeutung wie $R^5$ hat und Y ebenso wie
X O, S, SO oder $SO_2$ bedeutet und X und Y gleich oder verschieden sein können,
werden nach verschiedenen Verfahren hergestellt; sie eignen sich zur Behandlung von durch Viren bedingten Krankheiten.

EP 0 298 467 A2

# In 6- und 9-Stellung substituierte 2-Amino-8-aza-purine, ihre Verwendung, diese Purine enthaltende Arzneimittel und Verfahren zur Herstellung der Purine

In der DE-A 3.627.024 werden in 6- und 9-Stellung substituierte 2-Aminopurine offenbart. Ferner sind aus EP-A 201.289 in 6-Stellung substituierte 2-Amino-8-aza-purine bekannt, die in 9-Stellung einen 1,3-Dihydroxy-propoxymethyl-Rest tragen.

Die in beiden Druckschriften beschriebenen Purin-Derivate sollen gegen Viren - insbesondere gegen Herpes-Viren - wirksam sein.

Gegenstand der Erfindung sind nun Verbindungen der Formel 1 (s. Patentanspruch 1) und Arzneimittel, die sie als Wirkstoff enthalten, in denen

$R^1$ Wasserstoff, Halogen, Hydroxy, Mercapto, Azido, Amino oder Z-$R^5$ ist, wobei Z Sauerstoff, Schwefel, Sulfinyl, Sulfonyl oder NH und $R^5$ einen acyclischen aliphatischen Kohlenwasserstoffrest mit 1 bis 6 C-Atomen bedeutet, welcher a) unsubstituiert ist oder b) durch wenigstens eines der Merkmale modifiziert ist, daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl, das direkt oder über eine Alkylengruppe von 1 bis 3 C-Atomen an Z gebunden ist, oder Phenyl darstellt, wobei der Phenylring jeweils durch Halogen, Trifluormethyl, Alkoxy oder Alkylthio mit jeweils 1 bis 3 C-Atomen substituiert sein kann,

$R^2$ Wasserstoff ist oder die Bedeutung von $R^5$ hat oder eine Acylgruppe CO-$R^6$ ist, wobei $R^6$ Alkyl mit 1 bis 6 C-Atomen, Benzyl oder Phenyl ist,

$R^3$ Halogen, Azido, unsubstituiertes Amino oder den Rest Y-$R^4$ bedeutet, wobei $R^4$ dieselbe Bedeutung wie $R^5$ hat und X und Y Sauerstoff, Schwefel, Sulfinyl oder Sulfonyl bedeuten und gleich oder verschieden sein können.

Aliphatische Kohlenwasserstoffreste, Alkoxyreste und Alkylengruppen in den Phenylalkylresten können jeweils geradkettig oder verzweigt sein. Halogen bedeutet in den Resten $R^1$ und $R^3$ Fluor, Chlor, Brom oder Jod, im Rest $R^1$ bevorzugt Chlor oder Brom und im Rest $R^3$ bevorzugt Fluor oder Chlor. Im Rest $R^3$ haben die mindestens teilweise fluorierten Alkylreste vorzugsweise 1 bis 4, insbesondere 2 C-Atome (z.B. $-CH_2-CF_3$).

Bevorzugte Verbindungen sind diejenigen mit:

$R^1$ Wasserstoff, Hydroxy, Amino oder Z-$R^5$, wobei Z Sauerstoff und $R^5$ ein aliphatischer Kohlenwasserstoff mit 3 bis 5 Kohlenstoffatomen, insbesondere Isopropyl oder sek.-Butyl bedeutet, $R^2$ Wasserstoff oder eine Acylgruppe CO-$R^6$ ist, wobei $R^6$ Alkyl mit 1 bis 6 C-Atomen (insbesondere = $CH_3$), Benzyl oder Phenyl ist oder die Bedeutung von $R^5$ hat, $R^3$ Halogen, Azido, unsubstituiertes Amino oder den Rest Y-$R^4$ bedeutet, wobei X und Y Sauerstoff oder Schwefel bedeuten und gleich oder verschieden sein können und $R^4$ dieselbe Bedeutung wie $R^5$ hat.

Besonders bevorzugte Verbindungen sind die Verbindungen mit:

$R^1$ = H, OH oder $NH_2$,

$R^2$ = $C_3$-$C_5$-Alkyl, insbesondere i-$C_3H_7$ und sec. $C_4H_9$,

$R^3$ = $OR^4$, worin $R^4$ die gleiche Bedeutung wie $R^2$ besitzt,

und

X = O

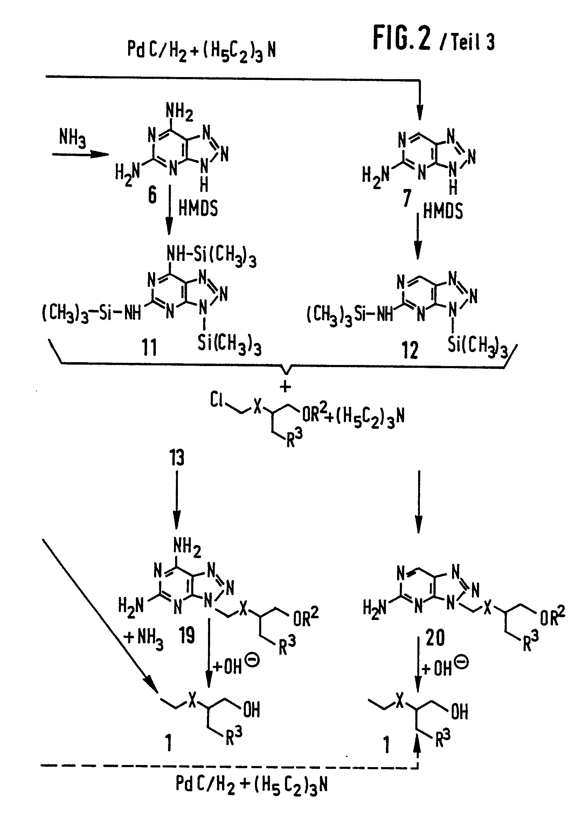

Die erfindungsgemäßen Verbindungen der allgemeinen Formel 1 können nach verschiedenen Methoden hergestellt werden, wobei man je nach Bedeutung des Restes $R^1$ verschiedene Ausgangsstoffe und geeignete Schutzgruppen wählt. Einige Ausführungsformen (A bis D) und die im folgenden angegebenen Formelbezeichnungen sind im Reaktionsschema (Figur 2) wiedergegeben, worin HMDS Hexamethyldisilazan, Pyr Pyridin und Ac Acetyl bedeutet und bei den Formeln, in denen $R^1$ OH, SH oder $HN_2$ bedeutet, auch eine Schreibweise in tautomerer Form gemäß formeln 2′, 3′ und 6′ (s. Figur 1) möglich wäre. In den Fällen, in denen mit Halogenverbindungen umgesetzt wird, ist das Halogen bevorzugt Chlor, wenngleich es auch ein anderes, wie Brom, sein kann.

Die als Ausgangsstoffe verwendeten Purine sind bekannt und überwiegend käuflich, bzw. nach bekannten Methoden darstellbar, so z.B. 2-Amino-8-aza-purin, 2-Amino-6-hydroxy-8-aza-purin und 2,6-Diamino-8-aza-purin aus 2,4,5-Triaminopyrimidin, 2,4,5-Triamino-6-hydroxy-pyrimidin und 2,4,5,6-Tetraamino-pyrimidin durch Diazotierung unter Ringschluß analog der Vorschrift in Org. Synth. Coll. Vol. 3, Seite 106. Die Ausgangsstoffe der Formel 13 und 14 sind überiegend bekannt - vgl. z.B. DE-A 3.627.024 - oder nach bekannten Methoden herstellbar.

**Verfahren A)**

Wenn $R^1$ Hydroxy (bzw. in der tautomeren Form Oxo) (Formel 2), Amino (bzw. Imino) (Formel 6), Wasserstoff (Formel 7) oder $R^1$ = Z-$R^5$ (Formel 21) - s. jeweils Figur 2- ist, setzt man zweckmäßig 2,6,9-Tris-(trimethylsilyl)- bzw. 2,9-Bis-(trimethylsilyl)-2-amino-8-aza-purine gemäß einer der Formeln 8, 11 und 12 mit Halogen-, insbesondere Chlorverbindungen der Formel 13 um, worin $R^2$ = Acyl ist und X und $R^3$ die angegebene Bedeutung haben, spaltet die Trimethylsilyl-Schutzgruppen ab und erhält so in 9-Position substituierte Verbindungen der Formel 1, speziell der Formeln 15, 19, 20 und 22. Dabei zeichnet sich in Formel 13 das in α-Stellung zu X stehende Halogen, insbesondere Chlor durch eine besondere Reaktivität aus. Natürlich kann man anstelle der Trimethylsilylschutzgruppe auch andere Trialkylsilylschutzgruppen verwenden, in denen der Alkylrest z.B. 1 bis 6, vorzugsweise 1 bis 3 C-Atome hat und geradkettig oder verzweigt ist.

Die erfindungsgemäßen Verbindungen 15, 19 und 20 werden gegebenenfalls zu anderen Verbindungen der Formel 1, in denen $R^2$ statt Acyl Wasserstoff ist, verseift (s. Figur 2).

**Verfahren B)**

Wenn $R^1$ Hydroxy (bzw. Oxo) (Formel 9), Mercapto (bzw. Thio) (Formel 10), Amino (bzw. Imino) (Formel 6a) oder Wasserstoff (Formel 7a) - s. jeweils Figur 1 ist, setzt man entsprechende Di- bzw. Triacyl-Verbindungen der Formeln 9, 10, 6a bzw. 7a mit reaktiven Acetxy-Verbindungen der Formel 14 um, worin $R^2$ Acyl ist und X und $R^3$ die angegebene Bedeutung haben. Man erhält so in 9-Position substitueirte Verbindungen der Formeln 16, 17, 6b und 7b. Diese erfindungsgemäßen Verbindungen werden gegebenenfalls zu anderen Verbindungen der Formel 1 verseift, in denen $R^2$ Wasserstoff ist.

**Verfahren C)**

Wenn $R^1$ Halogen, insbesondere Chlor ist (Formel 5), kann man $C_1$) diese Verbindung mit aktiven Halogenverbindungen der Formel 13 zu in 9-Position substituierten Verbindungen der Formel 18 umsetzen und diese $C_2$) gegebenenfalls zu anderen Verbindungen der Formel 1, in denen $R^2$ H ist, verseifen, oder $C_3$) vor oder nach der Verseifung zur Herstellung anderer Verbindungen der Formel 1 das Halogen austauschen, z.B. wenn

$R^1$ Hydroxy (bzw. Oxo) sein soll, durch saure Verseifung, z.B. mit wäßriger Salzsäure
$R^1$ Mercapto (bzw. Thio) sein soll, durch Umsetzung mit Schwefelwasserstoff,
$R^1$ Azido sein soll, durch Umsetzung mit einem Azid,
$R^1$ Amino (bzw. Imino) sein soll, durch Umsetzung mit Ammoniak,
$R^1$ Wasserstoff sein soll, durch katalytische dehalogenierende Hydrierung, z.B. mit Palladium-Kohle/Wasserstoff + Triäthylamin,
$R^1$ Z$R^5$ sein soll, durch Umsetzung mit Alkohlen bzw. Phenolen, Mercaptanen bzw. Thiophenolen und Aminen bzw. Anilinen,

wobei $R^2$ Acyl oder gegebenenfalls nach Verseifung Wasserstoff ist und $R^3$ und X die angegebene Bedeutung haben.

Bevorzugt werden nach Verfahren C Verbindungen hergestellt, in denen $R^1$ H, OH oder $NH_2$, $R^2$ Acyl, insbesondere Acetyl, und X O oder S sind, wobei $R^3$ und damit auch Y und $R^4$ die im Anspruch 1 angegebene Bedeutung haben.

Die Umsetzungen nach den Verfahren A bis C und dem weiter unten genannten Verfahren D, die Analogieverfahren darstellen, werden unter den üblichen Bedingungen durchgeführt. Bei den Verfahren A) bis C) liegen die Reaktionstemperaturen im allgemeinen zwischen etwa 0 und 150 °C, vorzugsweise zwischen etwa 50 und 100 °C. Gewöhnlich arbeitet man bei Normaldruck, jedoch ist es auch möglich, bei erhöhtem oder vermindertem Druck zu arbeiten, wenngleich hiermit normalerweise keine Vorteile verbunden sind. Die Lösungs- und Verteilungsmittel können natürlich sowohl in Form einheitlicher Stoffe als auch in Form von Gemischen verwendet werden. Zu den einzelnen Verfahren wird noch folgendes ausgeführt.

Die Umsetzungen nach Verfahren A) werden zweckmäßig in einem Lösungs- oder Verteilungsmittel durchgeführt. Dafür kommen insbesondere aprotische Lösungsmittel in Frage, wie Benzol, Toluol, die verschiedenen Xylole, ferner 1,2-Dichloräthan, Chlorbenzol, 1,2-Dimethoxyäthan, Dioxan. Zum Abfangen der gebildeten Halogenwasserstoffsäure empfiehlt sich die Verwendung einer Base, wie Triäthylamin oder N-Äthylmorpholin. Die Reaktionszeiten betragem je nach Reaktionstemperatur und Reaktionspartner einige

Minuten bis mehrere Stunden, meistens etwa 12-24 Stunden.

Wegen der Feuchtigkeitsempfindlichkeit der als Ausgangsstoffe verwendeten trialkylsilyl-geschützten Purine 8, 11, 12 empfiehlt sich die Verwendung eines Schutzgases im Reaktionsgefäß, z.B. Stickstoff oder Argon. Die Abspaltung der Trialkylsilyl-Schutzgruppen kann z.B. durch Kochen der Zwischenverbindung mit einem Alkohol, wie Methanol oder Äthanol erfolgen.

Die Umsetzungen nach Verfahren B) werden zweckmäßig ebenfalls in einem Lösungs- oder Verteilungsmittel durchgeführt. Dafür kommen insbesondere Sulfone als aprotische Lösungsmittel in Frage, wie Sulfolan (Tetramethylensulfon) oder Dipropylsulfon. Bei dieser Umsetzung empfiehlt sich die Verwendung eines sauren Katalysators, wie Methan-, Äthan-, Benzol- oder Toluolsulfonsäure. Die Reaktionszeiten betragen je nach Reaktionstemperatur und Reaktionspartner meist mehrere Stunden bis zu einigen Tagen.

Die Umsetzungen nach Verfahren $C_1$ werden ebenfalls zweckmäßig in einem Lösungs- oder Verteilungsmittel durchgeführt. Dafür kommen insbesondere polare, aprotische Lösungsmittel in Frage, wie Dimethylformamid, Dimethylacetamid, Tetramethylharnstoff, Hexamethylphosphorsäuretriamid oder Dimethylsulfoxid. Zum Abfangen der gebildeten Halogenwasserstoffsäure empfiehlt sich die Verwendung einer Base, z.B. der weiter unten bei der Verseifung genannten, wie Triäthylamin; N-Äthylmorpholin oder Alkalicarbonate, wie Natrium- oder Kaliumcarbonat. Die Reaktionszeiten betragen je nach Reaktionstemperatur und Reaktionspartner mehrere Minuten bis mehrere Stunden.

Der Halogenaustausch nach Verfahren $C_3$ wird z.B auf folgende Weise durchgeführt:

$C_3$a) Wenn $R^1$ Hydroxy (bzw. Oxo) ist, kann die Verseifung der Verbindung 18, z.B. mit 1-molarer wäßriger Salzsäure, durch 2-3-stündiges Kochen unter Rückfluß zur Verbindung 15 erfolgen; derartige Reaktionsbedingungen sind, ebenso wie die für das Verfahren $C_1$), eingehend in der Internationalen Anmeldung PCT/SE83/00254 beschrieben.

$C_3$b) Wenn $R^1$ Mercapto (bzw. Thio) ist, kann die Umsetzung der Verbindung 18 mit Schwefelwasserstoffgas in Gegenwart einer Base, wie Triäthylamin oder Pyridin, die gleichzeitig als Lösungsmittel dienen kann, oder in einem Alkohol wie Methanol, Äthanol, einem der Propanole oder Butanole, Äthylenglykolmonomethyläther oder - äthyläther, z.B. bei etwa 20-60 °C, zur Verbindung 17, erfolgen.

$C_3$c) Wenn $R^1$ Azido ist, kann die Umsetzung mit Alkaliaziden in polaren, eventuell auch in unpolaren, gegenüber den Reaktionsteilnehmern inerten Lösungsmitteln wie Dimethylform- bzw. -acetamid, Dimethylsulfoxyd, Hexamethylphosphorsäuretriamid, N-Methylpyrrolidon, Acetonitril und Tetramethylharnstoff unter üblichen Bedingungen, z.B zwischen etwa 20 und 120, vorzugsweise zwischen etwa 60 und 90 °C erfolgen.

$C_3$d) Wenn $R^1$ Amino (bzw. Imino) ist, kann die Umsetzung der Verbindung 18 z.B. mit Ammoniak (flüssig oder in alkoholischer Lösung), gegebenenfalls unter Verwendung eines gegenüber den Reaktionsteilnehmern inerten Lösungsmittels, wie Äthanol, im allgemeinen bei Temperaturen zwischen etwa 20 und 120 °C, vorzugsweise etwa 50-100 °C erfolgen, z.B. innerhalb von etwa 12 Stunden.

$C_3$e) Wenn $R^1$ Wasserstoff ist, kann die dehalogenierende Hydrierung der Verbindung 18 vorteilhaft mit Palladium-Kohle/Wasserstoff unter atmosphärischem Druck in Gegenwart einer hierfür geeigneten Base, wie Triäthylamin, unter üblichen Bedingungen, z.B. in etwa 12 Stunden bei Raumtemperatur, zur Verbindung 20 durchgeführt werden.

$C_3$f) Wenn $R^1$ Z-$R^5$ ist, Z Sauerstoff Schwefel oder NH ist und $R^5$ die angegebene Bedeutung haben, kann die Umsetzung der Verbindung 18 mit entsprachenden Hydroxy-, Mercapto- oder Aminoverbindungen erfolgen. Die Hydroxy- oder Mercaptoverbindungen werden in Form ihrer Alkalisalze, die Aminoverbindungen in Gegenwart einer anorganischen oder vorzugsweise organischen Base, wie Triäthylamin oder Pyridin, zur Umsetzung gebracht. Als Lösungsmittel kann bei den Hydroxyverbindungen eingesetzter Alkohol im Überschuß, bei Aminoverbindungen z.B. die Hilfsbase Pyridin verwendet werden. Im allgemeinen verwendet man als Lösungsmittel Alkohole, wie Propanole und Butanole, Alkylenglykole oder ihre Mono- bzw. Dialkyläther. Die Temperaturen liegen im allgemeinen zwischen etwa 25 und 130 °C, vorzugsweise zwischen etwa 80 und 100 °C. Die Reaktionszeiten liegen je nach Temperatur und Reaktionsteilnehmer meistens zwischen etwa 6 und 24 Stunden. Durch alaloge Umsetzung der Verbindung 3 bzw. 5 mit entsprechenden Hydroxy-, Mercapto- oder Aminoverbindungen und weitere Umsetzung mit Verbindung 13 bzw. 1 nach Verfahren A) lassen sich Verbindungen mit $R_1$ = Z-$R^5$ (Verbindung 21 bzw. 22) herstellen, wie in Figur 2 dargestellt.

**Verfahren D)**

Soweit nach einem der Verfahren A bis C erfindungsgemäße Verbindungen wie 15 bis 20, 6b und 7b erhalten werden , können diese, z.B. durch einwirkung von Basen, wie Ammoniak, Alkylaminen, wie Methyl- oder Äthylamin, den verschiedenen Propyl- oder Butylaminen, oder Alkalihydroxyden oder -carbonaten, wie

Natriumhydroxid, Kaliumhydroxid, Natriumcarbonat, Kaliumcarbonat in wäßriger oder alkoholischer (Methanol, Äthanol) Lösung, im allgemeinen bei Temperaturen von etwa 0-100° C, vorzugsweise bei etwa 20-50° C verseift werden.

Die meisten Verbindungen der Formeln 15 bis 19 fallen nach Verfahren A bis D als Gemisch von Purin-7- und -9-Isomeren in wechselnden Mengenverhältnissen an, die gegebenenfalls durch fraktionierte Kristallisation getrennt werden können. Im allgemeinen wird die Isomerentrennung mittels Säulenchromatographie z.B. über Kieselgel durchgeführt. Die Charakterisierung der Isomeren und ihre Prozentgehaltbestimmung erfolgt z.B durch Hochdruckflüssigkeitschromatographie (HPLC) unter Verwendung von Li-Chrosorb Si 60/5 µm der Firma Merck, Darmstadt, Deutschland.

Die erfindungsgemäßen Verbindungen besitzen, soweit nicht Y Sauerstoff und $R^2$ mit $R^4$ identisch ist, ein optisch aktives (chirales) Zentrum am Kohlenstoff-2-atom in der Ätherseitenkette. Die Verbindungen liegen als Racemate (DL-Form) vor. Eine Herstellung bzw. Isolierung der optisch aktiven Antipoden (Enantiomeren) ist möglich.

Die erfindungsgemäßen Verbindungen der Formel 1 besitzen wertvolle pharmazeutische Eigenschaften. Sie sind in vitro und in vivo gegen verschiedene Viren wirksam und eignen sich daher zu Bekämpfung verschiedener, durch DNS-Viren verursachter Krankheiten, wie Herpes, z.B. Typus simplex 1 und 2, Cytomegalie und RNS- Viren, z.B. Retroviren, sowie Autoimmunkrankheiten und zur Behandlung von Krebs. Arzneimittel, die sie enthalten, können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe un Streckmittel, Lösemittel, Emulgatoren, Gleitstoffe, Geschmackskorrigentien, Farbstoffe und/oder Puffersubstanzen in Frage. Als zweckmäßige Dosierung werden etwa 0,1-10, vorzugsweise etwa 0,2-8 mg/kg Körpergewicht verabreicht. Sie werden zweckmäßig in Dosierungseinheiten verabreicht, die mindestens die wirksame Tagesmenge der erfindungsgemäßen Verbindungen, z.B. etwa 30-300, vorzugsweise etwa 50-250 mg enthalten.

Die erfindungsgemäßen Verbindungen können auch in Kombination mit anderen Antivirusmitteln und Immunstimulantien, wie Interferonen verabreicht werden.

## Beispiele

1. 2-Amino-8-aza-9-[1,3-bis-(isopropoxy)-propyl-2-oxymethyl]-purin

6,8 g (0,05 Mol) 2-Amino-8-aza-purin (Fp. 287° C) und 1,7 g (0,0125 Mol) Ammoniumsulfat werden in 150 ml Hexamethyldisilazan eingetragen und unter Stickstoff-Schutzgas 3 Stunden zum Rückfluß (130° C) erhitzt. Nach Abkühlen wird das überschüssige Hexamethyldisilazan unter Wasserstrahlvakuum abdestilliert, der ölige Rückstand (2-Trimethylsilylamino-8-aza-9-trimethylsilylpurin) in 100 ml Toluol gelöst, 15 ml Triäthylamin zugegeben und unter Rühren und unter Stickstoff 18 g (0,08 Mol) 1,3-Bis-(isopropoxy)-2-chlormethoxy-propan, gelöst in etwas Toluol, bei Raumtemperatur zugetropft. Die Reaktionsmischung wird sodann 18 Stunden unter Rühren und Stickstoff auf 90° C erhitzt. Danach wird das Toluol unter Vakuum abgedampft, der ölige Rückstand in 250 ml Methanol gelöst und 20 Minuten unter Rückfluß erhitzt. Dabei werden die Trimethylsilylreste abgespalten. Das Methanol wird abgedampft, der Rückstand in 250 ml einer Mischung aus Essigester/n-Hexan 2:1 abgerührt, von unlöslichen Bestandteilen (Triäthylammonium-Hydrochlorid) abgesaugt und über eine Chromatographiesäule, beladen mit Kieselgel (Fa. Grace. Matrex LC 60A) mit gleichen Laufmittelgemisch gereinigt. Das Eluat wird eingedampft, der ölige Ruckstand durch Behandeln mit einer Mischung von n-Hexan und wenig Diisopropyläther zur Kristallisation gebracht. Die Kristalle werden abgesaugt, mit n-Hexan gewaschen und im Exsikkator getrocknet. Man erhält 6,3 g = 40 % der Theorie, weiße Kristalle, Fp. 71° C (93 % 9-Isomeres nach HPLC-analyse über Merck Li-Chrosorb Si 60/5 µm) $^1$H-NMR δ-Werte in Dimethylsulfoxid ($Me_2SO-d_6$, 60 MHz) : 7,35 (s, 2H - $NH_2$, C-2), 9,25 (s, 6H - H, C-6), 5,85 (s, 2H - $NCH_2O$), 3,7/3,8 (m, 1H - $OCH(CH_2)_2$), 3,3 (m, 4H -($CH(CH_2)_2$), 3,4 (m, 2H - [$OCH(CH_3)]_2$), 0,9/1,0 (d, 12H - [$OCH(CH_3)_2]_2$).

**Ausgangsstoffe:**

**2-Amino-8-aza-purin**

Darstellung durch Umsetzung von 2,4,5-Triamino-pyrimidin durch Diazotierung unter Ringschluß mittels Natriumnitrit in Eisessig bei 0-20 °C analog Vorschrift in Org. Synth. Coll. Vol. 3, Seite 106. Ausbeute: 76 % der Theorie, hellrosa Kristalle, Fp. 287 °C

**1,3-Bis-(isopropoxy)-2-chlormethoxy-propan**

Darstellung durch Umsetzung von 1,3-Bis-(isopropoxy)-2-hydroxypropan mit Formaldehyd und Chlorwasserstoffgas bei 0-5 °C nach Vorschrift in DE-A 3.627.024.

In gleicher Weise wie unter Beispiel 1 beschrieben wurden hergestellt:

**2. 2-Amino-6-hydroxy-8-aza-9[1,3-bis-(isopropoxy)-propyl-2-oxymethyl]-purin**

(aus 8-Aza-guanin und 1,3-Bis-(isopropoxy)-2-chlormethoxy-propan) weiße Kristalle, Fp. 196 °C (91 % 9-Isomeres nach HPLC-Analyse)
$^1$H-NMR δ-Werte in Dimethylsulfoxid ($Me_2SO-d_6$, 60 MHz) : 6,95 (s, 2H -$NH_2$, C-2), 11,0 (s, 1H -OH,C-6), 5,75 (s, 2H - $NCH_2O$), 3,7/3,8 (m, 1H - ($CH(CH_2)_2$), 3,4 (m, 4H -($CH(CH_2)_2$), 3,5 (m, 2H - [$OCH(CH_3)_2]_2$), 0,95/1,05 (d, 12H - [$OCH(CH_3)_2]_2$).

**3. 2,6-Diamino-8-aza-9-[1,3-bis-(isopropoxy)-propyl-2-oxymethyl]-purin**

(aus 2,6-Diamino-8-aza-purin und 1,3-Bis-(isopropoxy)-2 chlormethoxy-propan)
Weiße Kristalle, Fp. 185 °C
$^1$H-NMR-Werte in Dimethylsulfoxid ($Me_2SO-d_6$, 60 MHz): 6,45 (s, 2H -$NH_2$, C-2), 7,55 (s, 2H - $HN_2$, C-6), 5,75 (s, 2H -$NCH_2O$), 3,75 (m, 1H - ($OCH(CH_2)_2$ , 3,4 (m, 4H -($CH(CH_2)_2$), 3,5 (m, 2H - [$OCH(CH_3)_2]_2$), 0,95/1,05 (d, 12H - [$OCH(CH_3)_2]_2$).

**Ansprüche**

1. Verbindungen der Formel 1

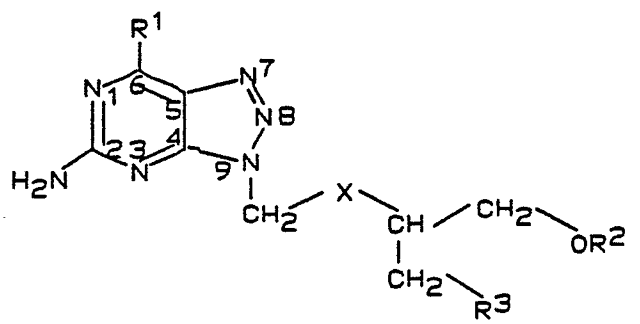

(1)

worin

$R^1$ H, Halogen OH, SH, Azido, $NH_2$ oder Z-$R^5$ ist ,wobei Z O, S, SO, $SO_2$ oder NH und $R^5$ einen acyclischen aliphatischen Kohlenwasserstoffrest mit 1 bis 6 C-Atomen bedeutet, welcher a) unsubstituiert ist oder b) durch wenigstens eines der Merkmale modifiziert ist, daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl, das direkt oder über eine Alkylengruppe von 1 bis 3 C-Atomen an Z gebunden ist, oder Phenyl darstellt, wobei der Phenylring jeweils durch Halogen, Trifluormethyl, Alkoxy oder Alkylthio mit jeweils 1 bis 3 C-Atomen substituiert sein kann,

$R^2$ H ist oder die Bedeutung von $R^5$ hat oder eine Acylgruppe CO-$R^6$ ist, wobei $R^6$ Alkyl mit 1 bis 6 C-Atomen, Benzyl oder Phenyl ist,

$R^3$ Halogen, Azido, unsubstituiertes Amino oder den Rest Y-$R^4$ bedeutet, wobei $R^4$ dieselbe Bedeutung wie $R^5$ hat und Y ebenso wie

X O, S, SO oder $SO_2$ bedeutet und X und Y gleich oder verschieden sein können.

2. Verbindungen nach Anspruch 1, dadurch gekennzeichnet, daß

$R^1$ = H, OH, $NH_2$ oder Z-$R^5$ (mit Z = O und $R^5$ = $C_3$-$C_5$-Alkyl, insbesondere i-$C_3H_7$ und sec. $C_4H_9$),

$R^2$ = H, Acyl $COR^6$ (mit $R^6$ = $C_1$-$C_6$-Alkyl, Benzyl oder Phenyl) oder die Bedeutung von $R^5$ hat,

$R^3$ = Halogen, $N_3$ oder Y-$R^4$ (mit Y=O oder S, und $R^4$ = gleiche Bedeutung wie $R^5$), und

X = O oder S.

3. Verbindungen nach Anspruch 1 oder 2, dadurch gekennzeichnet, daß

$R^1$ = H, OH oder $NH_2$,

$R^2$ = $C_3$-$C_5$-Alkyl, insbesondere i-$C_3H_7$ und sec. $C_4H_9$

$R^3$ = $OR^4$, worin $R^4$ die gleiche Bedeutung wie $R^2$ besitzt und

X = O.

4. Arzneimittel, gekennzeichnet durch einen Gehalt an einer wirksamen Menge wenigstens einer Verbindung gemäß einem oder mehreren der Ansprüche 1 bis 3.

5. Verfahren zur Herstellung von Verbindungen gemäß einem oder mehreren der Ansprüche 1 bis 3, dadurch gekennzeichnet, daß man unter üblichen Bedingungen

A) zur Herstellung von Verbindungen, in denen $R^1$ Hydroxy, Amino, Wasserstoff oder Z-$R^5$ ist, 2,6,9-Tris-(trialkylsilyl)- bzw. 2,9-Bis-(trialkylsilyl)-2-amino-8-aza-purine gemäß einer der Formeln 8, 11 und 12

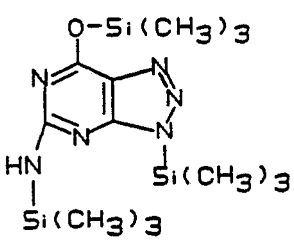
8

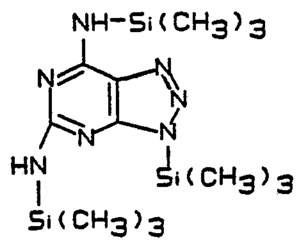
11

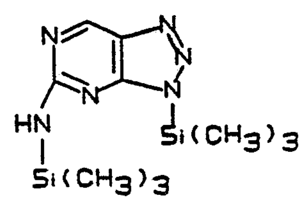
12

mit Halogen-, insbesondere Chlorverbindungen der Formel

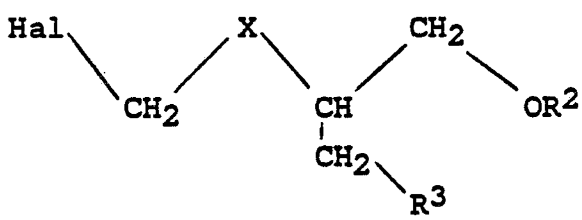
13

umsetzt, worin $R^2$ = Acyl ist und X und $R^3$ die in den Ansprüchen 1 bis 3 angegebene Bedeutung haben und die Trialkylsilylschutzgruppen abgespaltet oder

B) zur Herstellung von Verbindungen, in denen $R^1$ Hydroxy, Mercapto, Amino oder Wasserstoff ist, entsprechende Di-bzw. Triacyl -Verbindungen der Formeln 9, 10, 6a bzw. 7a

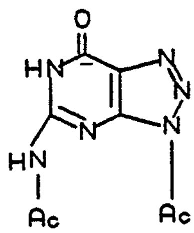

9

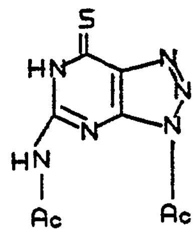

10

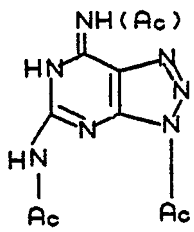

6a

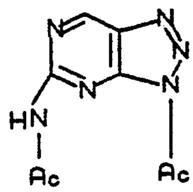

7a

mit reaktiven Acetoxy-Verbindungen der Formel 14

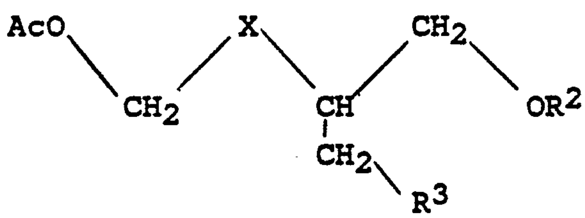

14

umsetzt, worin $R^2$ Acyl ist und X und $R^3$ die in den Ansprüchen 1 bis 3 angegebene Bedeutung haben oder

C) zur Herstellung von Verbindungen, in denen $R^1$ die in Formel 1 angebene Bedeutung besitzt, Verbindungen der Formel 5

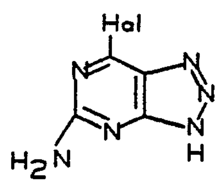

5

mit aktiven Halogen-Verbindungen der unter A angegebenen Formel 13 umsetzt und die so erhaltene Verbindung 18 isoliert oder durch saure Verseifung zu Verbindungen, in denen $R^1$ Hydroxy ist, oder durch Umsetzung mit Schwefelwasserstoff zu Verbindungen, in denen $R^1$ Mercapto ist, oder durch Umsetzung mit Alkaliaziden zu Verbindungen, in denen $R^1$ Azido ist, oder durch Umsetzung mit Ammoniak zu Verbindungen, in denen $R^1$ Amino ist, oder durch katalytische dehalogenierende Hydrierung zu Verbindungen, in denen $R^1$ Wasserstoff ist, umsetzt oder

D) zur Herstellung von Verbindungen, in denen $R^2$ Wasserstoff ist, eine Verbindung, die nach einem der Verfahren A bis C hergestellt ist, verseift.

6. Verwendung von Verbindungen gemäß einem oder mehreren der Ansprüche 1 bis 3 zur Behandlung von durch Viren verursachten Krankheiten.

FIG. 1

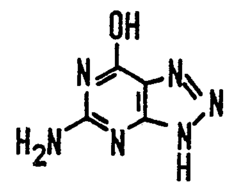

2'

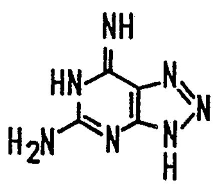

3'

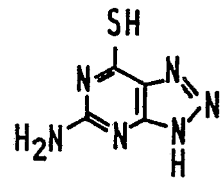

6'

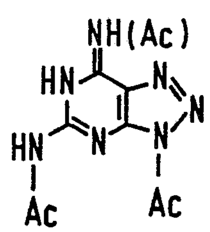

6a

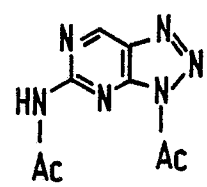

7a

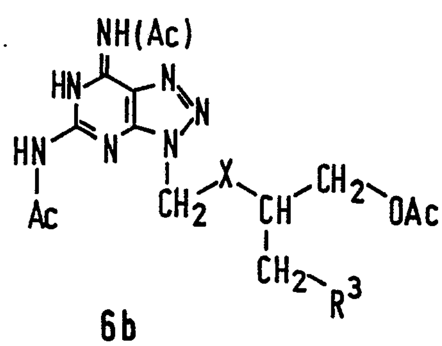

6b

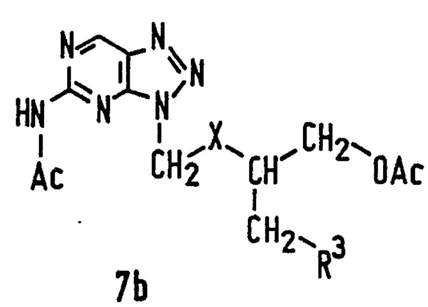

7b

FIG.2 / Teil 1

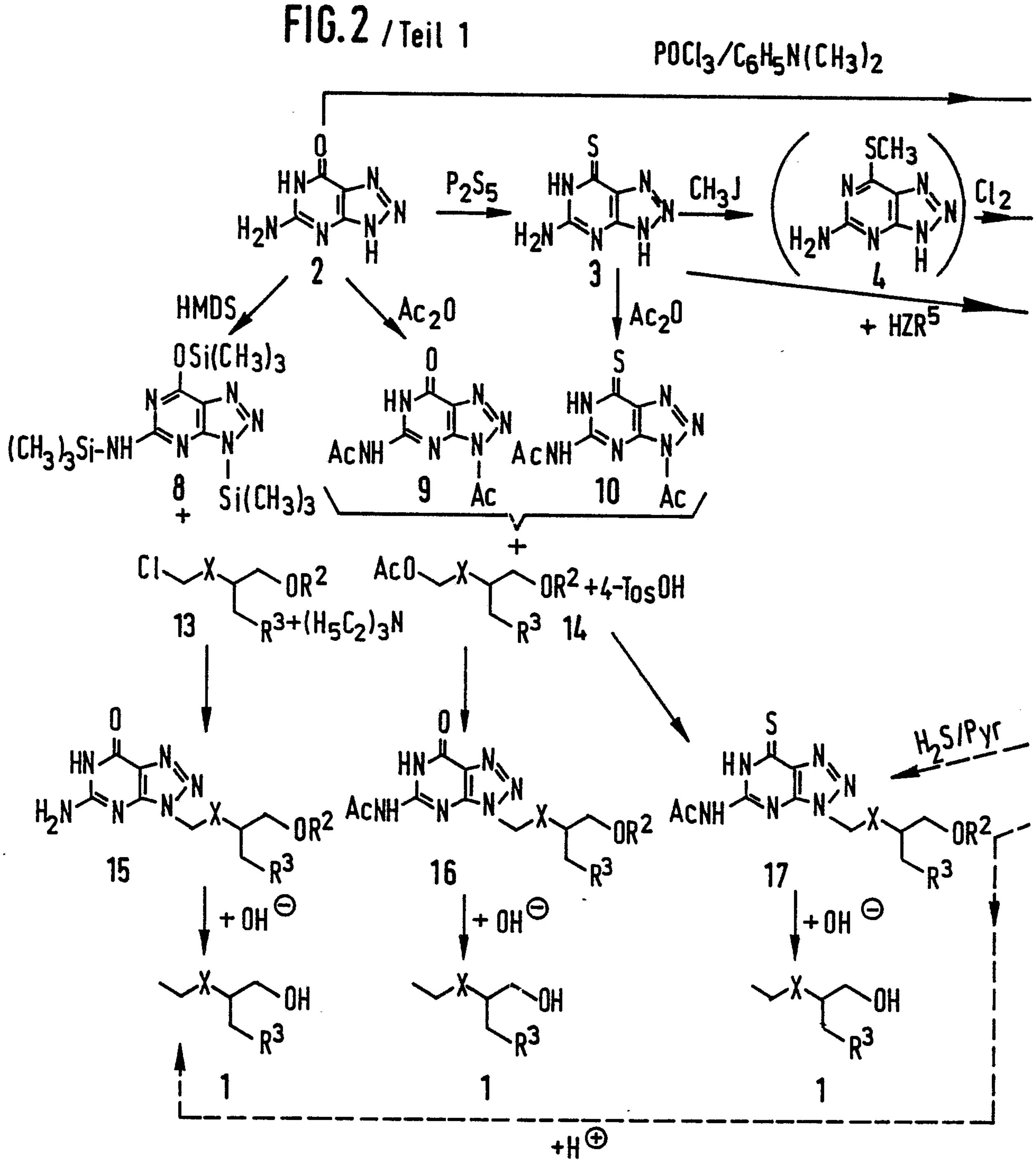

$POCl_3/C_6H_5N(CH_3)_2$
$P_2S_5$
$CH_3J$
$Cl_2$
HMDS
$Ac_2O$
$Ac_2O$
$+ HZR^5$
$+ 4\text{-TosOH}$
$+ (H_5C_2)_3N$
$H_2S/Pyr$
$+ OH^{\ominus}$
$+ OH^{\ominus}$
$+ OH^{\ominus}$
$+H^{\oplus}$
2
3
4
8
9
10
13
14
15
16
17
1
1
1

FIG. 2 / Teil 2

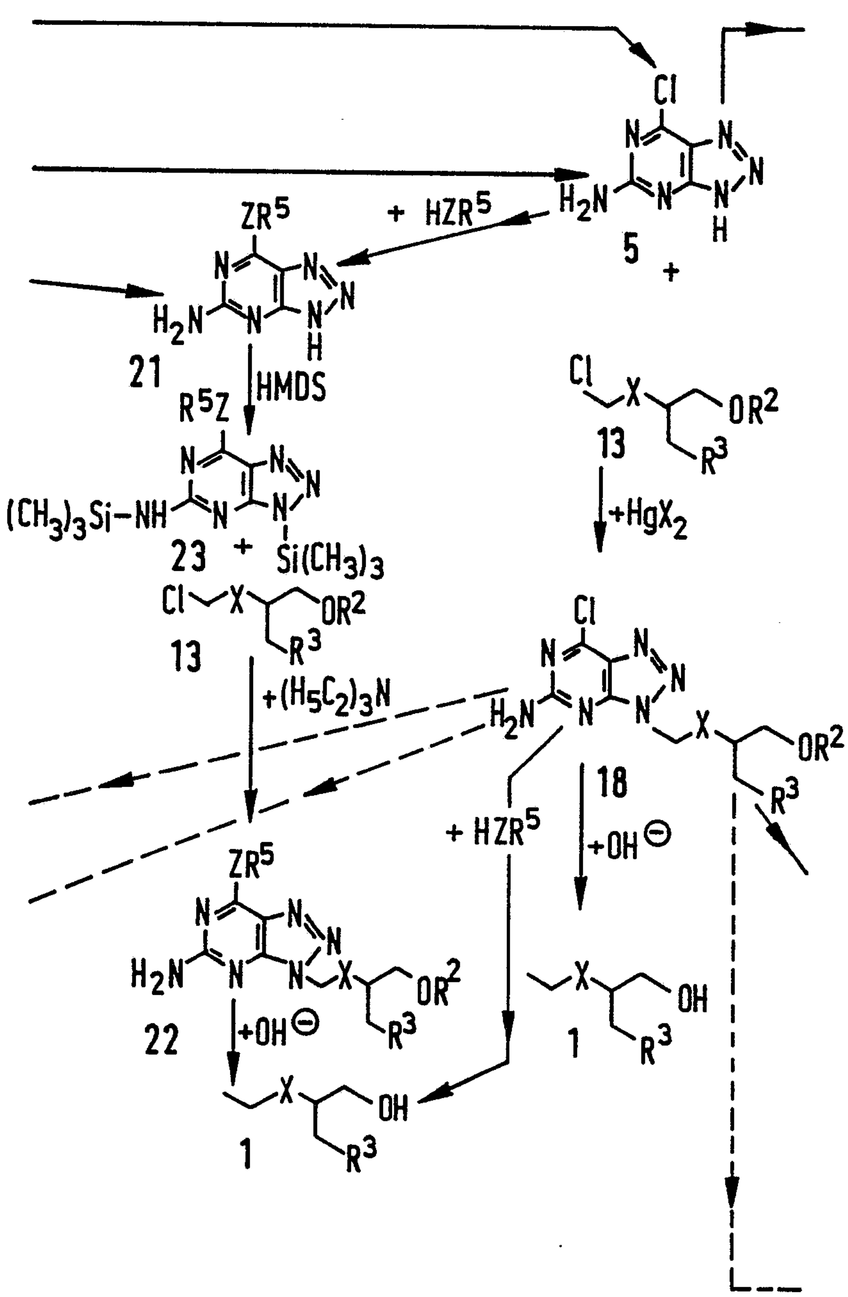

FIG. 2 / Teil 3

$Pd C/H_2 + (H_5C_2)_3 N$